(19) Europäisches Patentamt
European Patent Office
Office européen des brevets

(11) Veröffentlichungsnummer: **0 488 009 A2**

# (12) EUROPÄISCHE PATENTANMELDUNG

(21) Anmeldenummer: **91119625.1**

(22) Anmeldetag: **18.11.91**

(51) Int. Cl.5: **C07D 413/06**, A61K 31/535, C07D 417/06, C07D 417/14, A61K 31/54

(30) Priorität: **24.11.90 DE 4037426**

(43) Veröffentlichungstag der Anmeldung: **03.06.92 Patentblatt 92/23**

(84) Benannte Vertragsstaaten: **BE CH DE FR GB IT LI NL**

(71) Anmelder: **Kali-Chemie Pharma GmbH**
**Hans-Böckler-Allee 20 Postfach 220**
**W-3000 Hannover 1(DE)**

(72) Erfinder: **Jasserand, Daniel**
**65, rue Louis Blanc**
**F-69006 Lyon(FR)**
Erfinder: **Floc'h, Francois**
**1574, Chemin de Saint-André**
**F-69760 Limonest(FR)**
Erfinder: **White, Richard**
**16-18 Rue de Fenille**
**F-01000 Bourg en Bresse(FR)**

(74) Vertreter: **Lauer, Dieter, Dr.**
**c/o Solvay Deutschland GmbH, Postfach 220**
**W-3000 Hannover(DE)**

(54) **Piperidinoalkylbenzoxazin- und -thiazin-Verbindungen sowie Verfahren zur ihrer Herstellung und diese Verbindungen enthaltende Arzneimittel.**

(57) Es werden neue pharmakologisch wirksame Verbindungen der allgemeinen Formel I

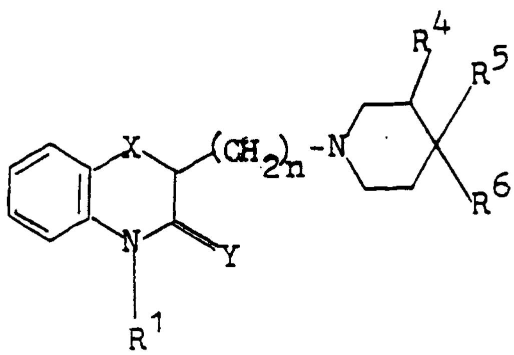

welche gegebenenfalls im Benzolring substituiert sind und worin

| | |
|---|---|
| X | Sauerstoff oder Schwefel bedeutet, |
| Y | Sauerstoff oder Schwefel bedeutet, |
| $R^1$ | Wasserstoff oder niederes Alkyl bedeutet, |
| n | für eine ganze Zahl von 0 bis 4 steht, |
| $R^4$ | Wasserstoff bedeutet, und |
| $R^5$ | Wasserstoff, Hydroxy oder Cyano bedeutet oder |
| $R^4$ und $R^5$ | gemeinsam eine Bindung bilden, |
| $R^6$ | für eine A-$R^7$-Gruppe steht, worin $R^7$ eine gegebenenfalls substituierte Phenylgruppe bedeutet, und A eine -$CH_2$-Gruppe, eine-CO-Gruppe oder eine Bindung bedeutet, oder |
| $R^6$ | für eine halogensubstituierte 1-Phenylmethyl-1H-benzimidazol-2-ylamino-Gruppe steht |

EP 0 488 009 A2

und deren physiologisch verträgliche Säureadditionssalze beschrieben.

Die vorliegende Erfindung betrifft neue, in 2-Stellung einen substituierten Piperidinoalkylrest tragende Benzoxazin- und Benzothiazin-3-on-Derivate und die entsprechenden 3-Thion-Derivate und deren Salze sowie diese Verbindungen enthaltende pharmazeutische Zubereitungen und Verfahren zur Herstellung dieser Verbindungen.

Aus der europäischen Patentanmeldung, Veröffentlichungs-Nr. 0 233 728, sind 1,4-Benzoxazin-3-on-Derivate bekannt, welche in 2-Stellung einen Phenylpiperazinoalkylrest tragen. Diese Verbindungen besitzen ausgeprägte hypotensive und vasodilatorische Wirkungen.

Der vorliegenden Erfindung liegt die Aufgabe zugrunde, neue als Antiallergica einsetzbare pharmazeutische Wirkstoffe zu entwickeln. Ferner liegt der Erfindung die Aufgabe zugrunde, neue Benzoxazin-Derivate mit wertvollen pharmakologischen Eigenschaften herzustellen.

Es wurde nun gefunden, daß die erfindungsgemäßen neuen heterocyclisch substituierten Verbindungen wertvolle pharmakologische Eigenschaften besitzen und antiinflammatorische und antiallergische Wirkungen zeigen und ein günstiges Wirkungsprofil mit geringer Toxizität und guter Verträglichkeit aufweisen. Aufgrund ihres Wirkungsprofils eignen sich die erfindungsgemäßen Substanzen als antiinflammatorisch wirksame Wirkstoffe und Antiallergica zur Behandlung von entzündlichen und allergischen Erkrankungen.

Die vorliegende Erfindung betrifft daher neue Verbindungen der allgemeinen Formel I
(s. Formel I)
worin

| | |
|---|---|
| X | Sauerstoff oder Schwefel bedeutet, |
| Y | Sauerstoff oder Schwefel bedeutet, |
| R | $^1$ Wasserstoff oder niederes Alkyl bedeutet, |
| $R^2$ | Wasserstoff, niederes Alkyl, Halogen, niederes Alkoxy, Hydroxy, Nitro oder Trifluormethyl bedeutet, und |
| $R^3$ | Wasserstoff, niederes Alkyl, Halogen oder niederes Alkoxy bedeutet, oder |
| $R^2$ und $R^3$ | an benachbarte Kohlenstoffatome gebunden sind und gemeinsam eine Alkylendioxygruppe mit 1-2 Kohlenstoffatomen bedeuten, |
| n | für eine ganze Zahl von 0 bis 4 steht, |
| $R^4$ | Wasserstoff bedeutet, und |
| $R^5$ | Wasserstoff, Hydroxy oder Cyano bedeutet, oder |
| $R^4$ und $R^5$ | gemeinsam eine Bindung bilden, |
| $R^6$ | für eine A-$R^7$-Gruppe steht, worin $R^7$ eine gegebenenfalls durch 1-3 Substituenten aus der Gruppe niederes Alkyl, niederes Alkoxy, Halogen und Trifluormethyl substituierte Phenylgruppe bedeutet, und A eine -$CH_2$-Gruppe, eine-CO-Gruppe oder eine Bindung bedeutet, oder |
| $R^6$ | für die Gruppe der Formel a<br>(s. Formel a)<br>worin $R^8$ Halogen bedeutet, steht, |

und deren physiologisch verträgliche Säureadditionssalze.

Sofern in den Verbindungen der Formel I die Substituenten $R^2$ und $R^3$ sowie Substituenten im Rest $R^6$ niedere Alkylgruppen darstellen oder enthalten, können diese gerade oder verzweigt sein und insbesondere 1 - 4, vorzugsweise 1 - 2 Kohlenstoffatome enthalten und stellen insbesondere Methyl oder Methoxy dar. Sofern die Substituenten Halogen darstellen, kommen insbesondere Fluor, Chlor oder Brom, vorzugsweise Chlor, in Frage. Der Benzolring des Ringgerüstes kann zweckmäßig unsubstituiert sein. Sofern der Benzolring durch einen Substituenten $R^2$ oder auch zwei Substituenten $R^2$ und $R^3$ substituiert ist, eignen sich insbesondere niedere Alkylsubstituenten, beispielsweise Methylsubstituenten.

Der Substituent $R^1$ stellt zweckmäßig Wasserstoff dar. Falls $R^1$ niederes Alkyl bedeutet, kann dieses geradkettig oder verzweigt sein und 1 - 4, insbesondere 1 - 2 Kohlenstoffatome enthalten.

In den Verbindungen der Formel I steht n für 0 - 4. Als günstig hat sich insbesondere eine Alkylenkette mit 3 oder 4 Gliedern erwiesen.

Die Substituenten $R^4$ und $R^5$ des Piperidinringes können für Wasserstoff stehen. $R^4$ und $R^5$ können jedoch auch gemeinsam eine Bindung bilden oder $R^5$ kann für Hydroxy oder auch Cyano stehen.

Der Substituent $R^6$ kann für eine Gruppe A-$R^7$ oder für die Gruppe a) stehen. In der Gruppe A-$R^7$ stellt A eine Methylengruppe oder auch eine Bindung dar. A kann auch eine CO-Gruppe darstellen. $R^7$ steht vorzugsweise für eine unsubstituierte oder gegebenenfalls auch für eine durch niederes Alkoxy, niederes Alkyl oder Halogen substituierte Phenylgruppe. Als günstig erweist sich insbesondere ein gegebenenfalls durch niederes Alkyl substituierter Benzylrest $R^6$. Sofern $R^6$ eine Gruppe a) darstellt, kann deren Halogensubstituent $R^8$ insbesondere für Fluor, Chlor oder Brom, vorzugsweise Fluor, stehen. Vorzugsweise ist $R^8$ in 4-Stellung des Benzylrestes angeordnet.

Erfindungsgemäß werden die neuen Verbindungen der Formel I und deren Säureadditionssalze erhalten, indem man in an sich bekannter Weise

a) zur Herstellung von Verbindungen der allgemeinen Formel Ia
(s. Formel Ia)
worin X, $R^1$, $R^2$, $R^3$, n, $R^4$, $R^5$ und $R^6$ obige Bedeutung besitzen, Verbindungen der allgemeinen Formel II
(s. Formel II)
worin X, $R^1$, $R^3$ und n obige Bedeutung besitzen, und $R^{2'}$ die für $R^2$ angegebene Bedeutung besitzt, wobei jedoch eine Hydroxygruppe durch eine nachträglich abspaltbare Schutzgruppe geschützt ist, und L für einen aminolytisch abspaltbaren Rest steht, umsetzt mit Verbindungen der allgemeinen Formel III
(s. Formel III)
worin $R^4$, $R^5$ und $R^6$ obige Bedeutung besitzen, und anschließend eine allfällige Hydroxyschutzgruppe wieder abspaltet, oder

b) Verbindungen der allgemeinen Formel Ia in Verbindungen der allgemeinen Formel Ib
(s. Formel Ib)
worin X, $R^1$, $R^2$, $R^3$, n, $R^4$, $R^5$ und $R^6$ obige Bedeutung besitzen, überführt, und

gewünschtenfalls erhaltene Verbindungen der allgemeinen Formel I, worin $R^1$ Wasserstoff bedeutet, zu Verbindungen der allgemeinen Formel I, worin $R^1$ niederes Alkyl bedeutet, alkyliert und/oder in erhaltenen Verbindungen der allgemeinen Formel I, worin $R^2$ Methoxy bedeutet, die Methoxygruppe zur Hydroxygruppe spaltet, und gewünschtenfalls freie Verbindungen der Formel I in ihre Säureadditionssalze überführt oder die Säureadditionssalze in die freien Verbindungen der Formel I überführt.

Die Umsetzung von Verbindungen der Formel II mit Verbindungen der Formel III gemäß Verfahrensvariante a) kann nach an sich zur Alkylierung von Aminen üblichen Methoden ausgeführt werden.

Die Umsetzung wird zweckmäßigerweise in einem unter den Reaktionsbedingungen inerten organischen Lösungsmittel unter basischen Bedingungen durchgeführt.

Als aminolytisch abspaltbare Reste L in den Verbindungen der Formel II eignen sich Halogene wie Chlor, Brom oder Jod, vorzugsweise Brom oder Chlor, oder auch ein Acyloxyrest O-Z, worin Z einen niederen Alkanoylrest oder einen organischen Sulfonsäurerest darstellt, beispielsweise den Rest einer Niederalkansulfonsäure, wie zum Beispiel Methansulfonsäure, oder von aromatischen Sulfonsäuren, wie Benzolsulfonsäure oder durch niederes Alkyl oder durch Halogen substituierten Benzolsulfonsäuren, zum Beispiel Toluolsulfonsäuren oder Brombenzolsulfonsäuren. Als inerte organische Lösungsmittel eignen sich insbesondere aprotische Lösungsmittel, beispielsweise aromatische Kohlenwasserstoffe, wie Toluol, Xylol oder Benzol, cyclische Äther wie Dioxan, Dimethylformamid, oder niedere Alkanole wie Äthanol oder Gemische aus den vorgenannten Lösungsmitteln. Zweckmäßigerweise wird bei erhöhten Temperaturen, beispielsweise Temperaturen zwischen 50 und 150 °C, vorzugsweise Siedetemperatur des Lösungsmittels, gearbeitet. Zweckmäßigerweise wird die Reaktion unter Zusatz einer organischen oder anorganischen Base durchgeführt. Es kann jedoch auch ein Überschuß der Verbindung der Formel III verwendet und dieser als interne Base benutzt werden. Beispiele geeigneter organischer Basen sind tertiäre organische Amine, insbesondere tertiäre Niederalkylamine wie Triäthylamin, Tripropylamine, N-Niederalkylmorpholine oder N-Niederalkylpiperidine. Geeignete anorganische Basen sind insbesondere Alkalimetallcarbonate oder -bicarbonate. Die Reaktionszeit kann je nach Reaktionsbedingungen zwischen 2 und 8 Stunden betragen. Als Schutzgruppe für eine allfällige Hydroxygruppe $R^2$ können an sich bekannte Ätherschutzgruppen gewählt werden, welche anschließend auf an sich bekannte Weise solvolytisch oder hydrogenolytisch wieder abgespalten werden,z.B. niedere Alkyl- oder Benzylgruppen.

Die Umwandlung der 3-On-Gruppe der Verbindungen der Formel Ia in die 3-Thion-Gruppe der Verbindungen der Formel Ib gemaß Verfahrensvariante b) kann nach an sich zum Austausch von Sauerstoff gegen Schwefel in Oxo-Verbindungen üblichen Methoden erfolgen. So können die Verbindungen der Formel Ib auf an sich bekannte Weise hergestellt werden, beispielsweise durch Behandeln der Verbindungen der Formel Ia mit einem Phosphorpentasulfid (z.B. $P_4S_{10}$) oder auch nach der von Lawesson et al. beschriebenen Methode (s. Bull. Soc. Chim. Belg. 87, 525-534, (1978)) durch Umsetzen mit 2,4-bis-(Methoxyphenyl)-1,3-dithia-2,4-diphosphetan-2,4-disulfid der Formel IV
(s. Formel IV)
(= bekannt als Lawessons Reagenz). Die Deoxosulfurierung erfolgt zweckmäßig in einem unter den Reaktionsbedingungen inerten organischen Lösungsmittel, beispielsweise einem aromatischem Kohlenwasserstoff, wie Xylol oder Toluol, bei erhöhten Temperaturen, beispielsweise Temperaturen zwischen 50 und 150 °C, zweckmäßigerweise bei Siedetemperatur des Reaktionsgemisches. Nach beendeter Reaktion können die sulfurierten Verbindungen von den Phosphorderivaten durch Filtration getrennt werden.

Erhaltene Verbindungen der Formel I, worin $R^1$ Wasserstoff bedeutet, können gewünschtenfalls nachträglich in an sich bekannter Weise zu den entsprechenden N-Alkylverbindungen alkyliert werden. Als

Alkylierungsmittel kommen Alkylhalogenide, insbesondere -jodide, Alkylsulfate oder Alkylsulfonsäureester in Frage. Zweckmäßigerweise wird die eine Amid- bzw. Thiamidgruppierung enthaltende Verbindung der Formel I zunächst mit einer starken Base wie beispielsweise einem Alkalimetallhydrid, -amid oder -alkoholat in einem inerten polaren organischen Lösungsmittel umgesetzt und anschließend weiter mit dem Alkylierungsmittel umgesetzt. Die Umsetzung kann bei einer Temperatur von 0 °C bis zur Siedetemperatur des Lösungsmittels erfolgen. Je nach dem verwendeten Metallierungsmittel eignen sich als Lösungsmittel Dimethylformamid oder cyclische Äther wie Tetrahydrofuran oder Dioxan, oder, falls die Base ein Metallalkoholat ist, auch die entsprechenden Alkohole. So kann die Umsetzung z.B. zweckmäßig in Dimethylformamid unter Verwendung von Natriumhydrid erfolgen.

In Verbindungen der Formel I, worin $R^2$ Methoxy bedeutet, kann die Methoxygruppe mit zur Spaltung von Methoxyaryläthern geeigneten Methoden auf an sich bekannte Weise zur Hydroxygruppe gespalten werden. Beispielsweise kann die Ätherspaltung durch Behandeln mit Jodwasserstoff in einem unter den Reaktionsbedingungen inerten Lösungsmittel, z.B. Acetanhydrid, oder mit Jodtrimethylsilan oder Bortribromid erfolgen.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit anorganischen Säuren, z.B. Halogenwasserstoffsäuren, insbesondere Chlorwasserstoffsäure, Schwefelsäure oder Phosphorsäuren oder mit organischen Säuren, beispielsweise niederen aliphatischen Mono- oder Dicarbonsäuren wie Maleinsäure, Fumarsäure, Milchsäure, Weinsäure oder Essigsaure, oder Sulfonsäuren, beispielsweise Niederalkylsulfonsäuren, wie Methansulfonsäure oder gegebenenfalls im Benzolring durch Halogen oder niederes Alkyl substituierte Benzolsulfonsäuren, wie p-Toluolsulfonsäure, oder Cyclohexylaminsulfonsäure.

Die Verbindungen der Formel I enthalten ein Chiralitätszentrum in Position 2 des Benzoxazin bzw. -thiazingerüstes und können in mehreren optisch aktiven enantiomeren Formen oder als Racemat vorliegen. Die vorliegende Erfindung umfaßt sowohl die racemischen Gemische wie auch die reinen optischen Isomeren der Verbindungen der Formel I.

Falls bei der Synthese Racemate der Verbindungen der Formel II eingesetzt werden, werden die Verbindungen der Formel I in Form von Racematen erhalten. Ausgehend von optisch aktiven Formen der Verbindungen der Formel II können optisch aktive Verbindungen der Formel I erhalten werden. Die optisch aktiven Verbindungen der Formel I können aus den racemischen Gemischen in an sich bekannter Weise erhalten werden, z.B. durch chromatographische Trennung an chiralen Trennmaterialien oder durch Umsetzung mit geeigneten optisch aktiven Säuren, beispielsweise Weinsäure oder 10-Camphersulfonsäure, und anschließende Auftrennung in ihre optisch aktiven Antipoden durch fraktionierte Kristallisation der gewonnenen Salze.

Die Ausgangsverbindungen der Formel II können ausgehend von 2-Aminophenolderivaten der Formel V
(s. Formel V)
worin $R^1$, $R^{2'}$, $R^3$ und X obige Bedeutung besitzen, erhalten werden.

So können die Verbindungen der Formel V auf an sich bekannte Weise mit einem $\beta$-Brom-acylbromid der Formel VI
(s. Formel VI)
worin L obige Bedeutung besitzt und n' 1 - 4 bedeutet, kondensiert werden zu Verbindungen der Formel IIa
(s. Formel IIa)
worin $R^1$, $R^{2'}$, $R^3$, X, n' und L obige Bedeutung besitzen. Die Kondensation kann in einem unter den Reaktionsbedingungen inerten Lösungsmittel, z.B. einem halogenierten Kohlenwasserstoff wie Chloroform, in Gegenwart einer Base, beispielsweise Alkalimetallhydrogencarbonaten oder -carbonaten, erfolgen und wird zweckmäßigerweise in Gegenwart eines Transferkatalysators, beispielsweise Bensyltrimethylammoniumchlorid, durchgeführt. Die Verbindungen der Formel V können auch mit $\beta$-Brom-alkancarbonsäuremethylestern der Formel VII
(s. Formel VII)
worin n' und L obige Bedeutung besitzen, zu Verbindungen der Formel IIa auf an sich bekannte Weise umgesetzt werden. Die Umsetzung kann beispielsweise in Dimethylformamid in Gegenwart einer anorganischen Base, beispielsweise eines Alkalimetallcarbonates, durchgeführt werden.

Verbindungen der allgemeinen Formel Va
(s. Formel Va)
worin $R^1$, $R^{2'}$und $R^3$ obige Bedeutung besitzen, sind bekannt oder können auf an sich bekannte Weise

ausgehend von Verbindungen der allgemeinen Formel IX
(s. Formel IX)
worin $R^{2'}$und $R^3$ obige Bedeutung besitzen, erhalten werden. Verbindungen der Formel IX können zur Einführung eines Alkylrestes $R^1$ auf an sich bekannte Weise alkyliert werden zu Verbindungen der allgemeinen Formel X
(s. Formel X)
worin $R^{2'}$ und $R^3$ obige Bedeutung besitzen und $R^{1'}$ niederes Alkyl bedeutet.

Verbindungen der Formeln IX und X können auf an sich bekannte Weise durch thermische Spaltung in wäßrigem alkalischem Medium, beispielsweise durch Kochen in Alkalimetallhydroxidlösung, in Verbindungen der Formel Va überführt werden.

Verbindungen der allgemeinen Formel Vb
(s. Formel Vb)
worin $R^1$, $R^{2'}$und $R^3$ obige Bedeutung besitzen, sind bekannt oder können nach an sich bekannten Methoden oder analog zu an sich bekannten Methoden erhalten werden. 2-Alkylaminophenol-Verbindungen ($R^1$ = niederes Alkyl) können ausgehend von den entsprechenden 2-Aminophenol-Verbindungen erhalten werden. Hierzu werden diese zunächst acyliert, wobei sowohl die phenolische Hydroxygruppe als auch die Aminogruppe mit einer Acylschutzgruppe versehen werden. In den erhaltenen Esteramid-Verbindungen wird die Amidgruppierung auf an sich bekannte Weise alkyliert, indem die Verbindungen mit einem Alkylierungsmittel, z.B. einem niederen Alkylhalogenid, -sulfat oder -sulfonat, in Gegenwart einer starken Base, beispielsweise einem Alkalimetallhydrid oder -hydroxid, umgesetzt werden, gegebenenfalls in Gegenwart eines Transferkatalysators, beispielsweise Benzyltrimethylammoniumchlorid. Die Umsetzung kann beispielsweise unter den vorstehend für die nachträgliche Alkylierung von Verbindungen der Formel I angegebenen Bedingungen erfolgen. Nach erfolgter Alkylierung können dann die Acylschutzgruppen auf an sich bekannte Weise durch saure oder alkalische Hydrolyse wieder abgespalten werden.

Verbindungen der Formel II, worin n für 0 steht, können ausgehend von Verbindungen der Formel VIII
(s. Formel VIII)
worin $R^1$, $R^{2'}$, $R^3$ und X obige Bedeutung besitzen, erhalten werden. So kann man zur Herstellung von Verbindungen der Formel IIb
(s. Formel IIb)
worin $R^1$, $R^{2'}$ und $R^3$ obige Bedeutung besitzen und L' Halogen bedeutet, in Verbindungen der Formel VIII auf an sich bekannte Weise einen Halogensubstituenten L', insbesondere Chlor, einführen durch Behandlung mit einem Halogenierungsmittel, beispielsweise Sulfurylchlorid. Die Chlorierung kann in einem unter den Reaktionsbedingungen inerten Lösungsmittel, beispielsweise einem halogenierten Kohlenwasserstoff wie Dichlormethan durchgeführt werden.

Verbindungen der Formel VIII sind bekannt oder können auf an sich bekannte Weise erhalten werden, beispielsweise indem man Verbindungen der Formel V mit Chloracetylchlorid kondensiert. Die Kondensation kann unter den zur Herstellung von Verbindungen der Formel IIa angegebenen Reaktionsbedingungen erfolgen.

Verbindungen der Formel III sind bekannt oder können nach an sich bekannten Methoden oder analog zu bekannten Methoden hergestellt werden. So können Verbindungen der Formel III beispielsweise ausgehend von Piperidon-Verbindungen der allgemeinen Formel XI
(s. Formel XI)
worin Z eine Aminoschutzgruppe bedeutet, erhalten werden. Hierzu werden die Aldehyde der Formel XI zunächst in einer Grignardreaktion mit einem Grignard-Reagenz der allgemeinen Formel XII
(s. Formel XII)
worin $R^6$ und L' obige Bedeutung besitzen, zu den Alkoholen der allgemeinen Formel XIII
(s. Formel XIII)
worin $R^6$ und Z obige Bedeutung besitzen, umgesetzt, und aus diesen durch Entfernung der Aminoschutzgruppe Verbindungen der Formel III, worin $R^5$ Hydroxy bedeutet, erhalten. Zu Verbindungen der Formel III, worin $R^4$ und $R^5$ gemeinsam eine Bindung darstellen, gelangt man durch Wasserabspaltung und Entfernung der Aminoschutzgruppe aus Verbindungen der Formel XIII. Zu Verbindungen der Formel III, worin $R^5$ Wasserstoff bedeutet, gelangt man durch Reduktion der Alkohole der Formel XIII, z.B. durch katalytische oder chemische Hydrogenolyse, und, gegebenenfalls gleichzeitiger, Abspaltung der Aminoschutzgruppe. Zur Herstellung von Verbindungen der Formel III, worin $R^5$ Cyano bedeutet, wird die Hydroxygruppe der Verbindungen der Formel XIII auf an sich bekannte Weise zunächst durch Umsetzung mit einem Halogenierungsmittel in Halogen überführt und dieses dann durch Umsetzen mit einem Cyanid in den Cyanrest überführt.

Zur Herstellung von Verbindungen der Formel III, worin $R^6$ für eine CO-$R^7$ Gruppe steht, können auch

Piperidincarbonsäurehalogenide der allgemeinen Formel XIV
(s. Formel XIV)
worin Z und L' obige Bedeutung besitzen, zunächst mit Verbindungen der allgemeinen Formel XV
(s. Formel XV)
worin $R^7$ obige Bedeutung besitzt, in einer Friedel-Crafts Reaktion in Gegenwart von Aluminiumchlorid umgesetzt werden zu Ketonen der allgemeinen Formel XVI
(s. Formel XVI)
worin Z und $R^7$ obige Bedeutung besitzen, und in diesen anschließend die Aminoschutzgruppe abgespalten werden.

Zur Herstellung von Verbindungen der Formel III, worin $R^6$ für die Gruppe a) steht, können auch Verbindungen der allgemeinen Formal XVII
(s. Formel XVII)
worin L' und $R^8$ obige Bedeutung besitzen, mit der Aminoverbindung der allgemeinen Formel XVIII
(s. Formel XVIII)
umgesetzt und anschließend die Äthoxycarbonyl-Schutzgruppe abgespalten werden.

Die Verbindungen der Formel I und ihre pharmakologisch akzeptablen Säureadditionssalze zeichnen sich durch interessante pharmakologische Eigenschaften aus und besitzen antiinflammatorische und antiallergische Wirkungen. Insbesondere zeigen die Verbindungen ein zur Behandlung von asthmatischen Beschwerden günstiges Wirkungsprofil mit geringer Toxizität und guter Verträglichkeit.

Asthma ist ein chronisches inflammatorisches Lungenleiden, welches durch episodisch auftretende reversible Behinderungen der Atemwege charakterisiert ist. Es wird allgemein angenommen, daß die Auslösung von asthmatischen Beschwerden und Anfällen von einem als Mastzelle bekannten parenchymalen und interstitialen Zelltyp ausgeht. Diese Mastzellen enthalten vorgebildete Entzündungsmediatoren und Spasmogene, insbesondere Histamin. Sie sind auch befähigt, eine Reihe von von Membranlipiden abstammenden Mediatoren neu zu synthetisieren. Mastzellen wirken auch im Verein mit einer Vielzahl von Begleitzellen, welche alle fähig sind, inflammatorische und pro-inflammatorische Mediatoren zu synthetisieren.

Solange keine allergieauslösenden Bedingungen vorliegen, befinden sich die Mastzellen in quasi unbeteiligter Wartestellung. Der Schlüssel zu allergischen Reaktionen liegt in der Anwesenheit hoher Konzentrationen von zirkulierenden IgE-Antikörpern. Wenn diese Antikörper an ein entsprechendes Antigen gebunden werden, aktivieren sie beides, sowohl die Degranulation und Freisetzung der vorgeformten Mediatoren als auch die Neusynthese anderer Mediatoren.

Da Asthma ein inflammatorisches obstruktives Lungenleiden ist, stützt sich die Therapie auf im wesentlichen zwei Wege: Erleichterung der symptomatischen Beschwerden durch Verabreichung von Bronchodilatoren wie $\beta$-Sympathicomimetica, Xanthin-Derivate und Anticholinergica; Verabreichung von antiinflammatorischen Wirkstoffen wie Dinatriumcromoglicinat und Steroiden; und gegen spezifische Mediatoren wie z. B. Histamin zielgerichtete Therapien. Eine Behandlung zur Linderung der symptomatischen Beschwerden ist bei etwa 50 % der Asthmatiker angemessen, trägt jedoch nichts dazu bei, die Ursachen, das ist die Entzündung, zu lindern. Antiinflammatorische Wirkstoffe können die Entzündung eindämmen, besitzen jedoch oft unerwünschte Nebenwirkungen und werden oft gleichzeitig mit Bronchodilatoren verabreicht. Auf einen spezifischen Mediator zielgerichtete Therapien sind allein völlig unzureichend, da es eine Vielzahl von Mediatoren gibt.

Die erfindungsgemäßen Substanzen zeichnen sich dadurch aus, daß sie antiinflammatorisch wirksam sind und zielgerichtet gegen einen oder mehrere der drei Mediatortypen Histamin, Leukotriene und Blutplättchenaggregationsfaktor, welche nicht nur bei akuten Bronchospasmen sondern auch bei der Aufrechterhaltung der chronischen Entzündung beteiligt sind, wirken oder auch über mediatorenspezifische Rezeptoren gegen die betreffenden Targetzellen wirksam sind.

Die antiinflammatorischen und antiallgergischen Eigenschaften der Verbindungen lassen sich in pharmakologischen Standardtestmethoden in vitro und in vivo nachweisen.

Beschreibung der Testmethoden

1. Bestimmung der Hemmung der passiven cutanen Anaphylaxie (P.C.A.) und der durch Histamin induzierten anaphylaktoiden cutanen Reaktion.

Die P.C.A.-Reaktion wird nach den von Goose et al (J.N. Immunology 16 (1969), 749) und von Martin et al (Arch. Pharmacol. 316 (1981), 186) beschriebenen Methoden durchgeführt.

Das in dem Test verwendete IgE-reiche Ovoalbumin-Antiserum wird in immunisierten Brown-Norway-Ratten gewonnen. Hierzu wird den Ratten zur Immunisierung eine i.p.-Injektion einer Mischung von 100 µg Ovoalbumin mit einer Bordetella-pertussis-Suspension (Vaxicoq[R], Hersteller: Institut Merieux, enthaltend 5 x $10^9$ Organismen und 1,25 mg $Al(OH)_3$) gegeben. Nach 20 Tagen erhalten die Tiere eine weitere i.p.-Injektion einer Lösung von 10 µg Ovoalbumin in 0,5 ml physiologischer Kochsalzlösung zur Reimmunisierung. Nach weiteren vier Tagen werden die Tiere entblutet und das Blut zentrifugiert. Das so erhaltene Antiserum wird bis zum Gebrauch bei -20 ° C gelagert.

Die Bestimmung der Hemmung der passiven cutanen Anaphylaxie und der durch Histamin induzierten anaphylaktoiden cutanen Reaktion wird wie folgt durchgeführt:
Zur passiven Sensibilisierung gegen Ovoalbumin werden Sprague-Dawley-Ratten mit einem Körpergewicht von 150-180 g 50 µl einer 1:75-Verdünnung des IgE-reichen Ovoalbumin-Antiserum in physiologischer Natriumchlorid-Lösung intradermal an einer Flanke injiziert.

24 Stunden nach der Sensibilisierung wird den Ratten zur Auslösung einer passiven cutanen Anaphylaxie eine Lösung von 8,25 mg/kg Ovoalbumin und 26,4 mg/kg eines blauen Farbstoffes (Evans's Blue) nach Martin et al. i.v. appliziert. Der Ovoalbumin-Reiz bewirkt eine lokale anaphylaktische Reaktion an der Stelle, an welcher das Antiserum injiziert worden war.

Zur Bestimmung der histamininduzierten anaphylaktoiden Hautreaktion werden den Tieren an der der Antiserumapplikation gegenüberliegenden Flanke direkt vor der iv-Injektion der das Ovoalbumin und den blauen Farbstoff enthaltenden Lösung 50 µl einer 0,8 mg/ml Histamin enthaltenden physiologischen Natriumchloridlösung intradermal injiziert.

Am Untersuchungstage werden die Testsubstanzen in destilliertem Wasser, welches 1 Vol.-% Dimethylformamid und 1 Vol.-% Tween[R]20 (= Polyoxyäthylen(20)sorbitanmonolaurat) enthält, gelöst. Eine Stunde vor der reizauslösenden Ovoalbumin-Applikation erhält jedes Tier 2 x $10^{-5}$ Mol/kg Testsubstanz jeweils in 0,5 ml Lösung oral appliziert. Eine Kontrollgruppe erhält zum Vergleich nur das Lösungsmittel.

Die durch die reizauslösende i.v.-Ovoalbumininjektion hervorgerufenen ödematösen anaphylactischen (P.C.A.) und anaphylactoiden (histamininduziert) Reaktionen, welche sich durch Ödembildung, Schwellung und Exsudation von blauem Farbstoff zeigen, werden 30 Minuten nach der Reizung durch die iv-Ovoalbumin-Injektion ausgewertet. Dies geschieht durch visuelle Bestimmung des Ausmaßes des Austritts von blauem Farbstoff an den Stellen der Ödembildung. Mit Hilfe von Vergleichsskalen wird die durch die Testsubstanzen hervorgerufene prozentuale Hemmung der anaphylaktischen und anaphylactoiden Reaktionen im Vergleich zu den Reaktionen der nicht mit Testsubstanz behandelten Kontrolltiere bestimmt.

Die nach den vorstehenden Testmethoden mit Verbindungen der Formel I erhaltenen Ergebnisse werden in der nachfolgenden Tabelle A wiedergegeben. Die für die Verbindungen der Formel I angegebenen Beispielsnummern beziehen sich auf die nachfolgenden Herstellungsbeispiele.

Tabelle A

| Testsubst. Bsp. Nr. | Hemmwirkung gegenüber cutanen anaphylactischen und anaphylactoiden Reaktionen in der Ratte % Hemmung bei Dosis von 2 x $10^{-5}$ Mol/kg p.o. | |
|---|---|---|
| | passive cutane Anaphylaxie (P.C.A.) | Histamin-induzierte anaphylactoide Reaktion |
| 12 | 35 | 20 |
| 20 | 20 | 40 |
| 1 | 85 | 85 |
| 8 | 100 | 100 |
| 10 | 55 | 30 |
| 11 | 45 | 20 |
| 15 | 30 | 10 |
| 18 | 90 | 50 |

2. Bestimmung der minimalen toxischen Dosis.

Männlichen Mäusen von 20-25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder stark toxische Symptome beobachtet werden, werden weiteren Mäusen zunehmend geringere Dosen verabreicht. Die

niedrigste Dosis, welche Tod oder starke toxische Symptome hervorruft, wird als minimale toxische Dosis in der nachstehenden Tabelle B angegeben.

Tabelle B

| Testsubstanz Beispiel Nr. | Minimale toxische Dosis mg/kg Maus p.o. |
|---|---|
| 3 | 300 |
| 4 | 300 |
| 8 | 300 |
| 10 | 300 |
| 15 | 300 |
| 16 | >300 |
| 21 | 100 |

3. Untersuchung der auf Histamin-($H_1$)-Rezeptor-Antagonismus beruhenden Antihistamin-($H_1$)-Wirkung in vitro.

Zur Untersuchung des Histamin-($H_1$)-Rezeptor-Antagonismus der Substanzen wird deren Hemmwirkung auf durch Histamin induzierte Kontraktionen der glatten Muskulatur am isolierten Organ in vitro bestimmt. Hierzu eignen sich isolierte Organstreifen aus dem Ileum. Sie reagieren in einem Organbad aus physiologischer Salzlösung auf Zugabe von Histamin mit Kontraktion. Durch Zugabe der erfindungsgemäßen Verbindungen wird diese durch Histaminzugabe induzierte Kontraktion der glatten Muskulatur der Ileumstreifen gemindert. Das Ausmaß der Rückbildung der Kontraktion ist ein Indiz für die Antihistamin-($H_1$)-Wirksamkeit der Verbindungen. Die Untersuchung wird der ursprünglich von Magnus (Pflügers Arch. 102, 123 (1904)) beschriebenen Methode analog durchgeführt.

Versuchsbeschreibung für die Bestimmung der Hemmwirkung auf die durch eine 5 x $10^{-6}$-molare Histaminkonzentration induzierte Kontraktion am isolierten glatten Muskel des Meerschweinchen-Ileums.

Für den Versuch werden 1,5 cm lange Segmente des Ileums von Dunkin Hartley-Meerschweinchen mit einem Körpergewicht von 300 - 500 g eingesetzt.

Jeder Streifen wird in ein Organbad aus 10 ml physiologischer Salzlösung nach Krebs-Henseleit gegeben und in einer zur isotonischen Messung von Längenänderungen des Ileum-Streifens üblichen Apparatur (automatisiertes Celaster-Meßgerät) so befestigt, daß das Gewebe unter 1 g Spannung steht. Das Bad wird bei einem pH-Wert von 7,4 gehalten und mit einer Mischung aus 5 % $CO_2$ und 95 % $O_2$ begast. Nach einer Äquilibrierungsphase wird eine isotonische Kontraktion des Gewebes hervorgerufen, indem Histamin in einer Endkonzentration von 5 x $10^{-6}$ Mol/l zugegeben und nach einer Kontaktzeit von 30 Sekunden wieder ausgewaschen wird. Nur solche Gewebe, bei welchen drei reproduzierbare Kontraktionen in 10-Minuten-Abständen erhalten werden, werden in dem nachfolgenden Test verwendet. Dann werden die Testsubstanzen in einer Endkonzentration von $10^{-6}$ Mol/l zugegeben, und nach 30 Sekunden Kontaktzeit wird wiederum Histamin bis zu einer Konzentration von 5 x $10^{-6}$ Mol/l zugegeben. Die auftretenden Kontraktionen werden über 30 Sekunden gemessen. Anschließend wird das Gewebe über einen Zeitraum von 10 Minuten mehrfach gewaschen. Dann wird nochmals durch Histaminzugabe ein Kontraktionsreiz ausgelöst. Die auftretenden Kontraktionen werden wiederum über 30 Sekunden gemessen. Der Unterschied zwischen der Amplitude der durch Histaminzugabe allein erhaltenen Kontraktion und der Amplitude der in Gegenwart der Testsubstanz erhaltenen Kontraktion wird bestimmt und als % Hemmung berechnet.

Die nachfolgende Tabelle C gibt die mit den Testsubstanzen nach der vorstehend beschriebenen Methode erhaltenen Ergebnisse wieder. In der Tabelle werden die Hemmwirkungen auf die 30 Sekunden nach Applikation der Testsubstanz durch Histamin induzierten Kontraktionen und auf die durch die 10 Minuten später erfolgte Histamingabe induzierten Kontraktionen angegeben.

Tabelle C

| Testsubst. Bsp. Nr. | in vitro ($H_1$)-Rezeptorantagonismus % Hemmwirkung auf histamin-induzierte Kontraktionen des Ileums bei Histaminkonzentration 5 x $10^{-6}$ Mol/l und Testsubstanzkonzentration $10^{-6}$ Mol/l | |
|---|---|---|
| | nach 30 sec. | nach 10 min. |
| 9 | 35 | 12 |
| 8 | 5 | 44 |
| 13 | 49 | 8 |

4. Bestimmung der Anti-P.A.F.-Wirkung in vitro.

P.A.F. (= Platelet Activating Factor = Blutplättchenaggregationsfaktor) ist ein phosphorlipidischer Mediator, welcher eine Vielzahl von Wirkungen besitzt. Die Aktivierung der Blutplättchenaggregation führt zur Induzierung langanhaltender Bronchokontraktionen und Überreaktivität der Luftwege.

In diesem Test wird nach der von Mikashima et al. beschriebenen Methode (Jap. J. Pharmacol. 44 - (1987) 387-391) die Wirkung der Testsubstanzen auf eine durch Zugabe von P.A.F. induzierte Blutplättchenaggregation in einer aus Kaninchenblut gewonnenen Blutplättchensuspension untersucht.

Es wird eine Suspension von aus Kaninchenblut stammenden Blutplättchen verwendet, welche 4 x $10^9$ Blutplättchen/ml in einer auf pH 7,4 eingestellten modifizierten Tyrode-Pufferlösung (= Tyrode-Lösung*mit Zusatz von 1,3 mM/l $CaCl_2$ und 2,5 g/l Gelatine) enthält. Die Blutplättchen werden aus 10 ml Blutproben von jeweils drei Kaninchen (Neuseelandhybriden, Körpergewicht 3 - 4 kg) erhalten. Hierzu werden die Blutproben mit Äthylendiamintetraessigsäure behandelt und nach der Methode von Artley et al. (Brit. J. Hämatol. 19 (1970), 7-17) gewaschen. Sodann wird durch Zentrifugieren (20 Minuten bei 400 x g) zunächst ein blutplättchenreiches Plasma abgetrennt. Durch nochmaliges Zentrifugieren für 15 Minuten bei 1400 x g werden die Blutplättchen von dem Plasma getrennt. Nach dem Zentrifugieren werden die als Sediment verbleibenden Blutplättchen in einer Tyrode-Pufferlösung (jedoch ohne Calcium) resuspendiert. Sodann werden 0,4 mMol Lysinacetylsalicylat zugegeben, und nach 15 Minuten werden die Blutplättchen nochmals sedimentiert. Das Sediment wird in der vorgenannten modifizierten Tyrodepufferlösung resuspendiert, und die Anzahl der Blutplättchen in der erhaltenen Suspension wird auf den gewünschten Gehalt eingestellt.

Es wird eine 40 x $10^{-9}$-molare P.A.F.-Lösung als Reagenz eingesetzt. Diese Lösung wird aus einer 1,8 x $10^{-3}$-molaren Vorratslösung in Chloroform hergestellt. Hierzu wird eine 10 µl Probe der Vorratslösung zur Trockne eingedampft und wieder in 180 µl der modifizierten Tyrodelösung, welcher 0,25 % von Lipiden befreites Rinderserum-Albumin zugesetzt worden war, gelöst. Daraus werden sodann $10^{-5}$-molare Arbeitslösungen hergestellt und gefroren aufbewahrt. Für Tests werden Proben dieser Lösungen entsprechend verdünnt.

Zur Testdurchführung werden in einer mit einem kleinen Magnetrührer versehenen Aggregationsröhre zu 330 µl der modifizierten Tyrode-Pufferlösung unter Rühren (1000 UpM) 50 µl der Blutplättchensuspension und 10 µl einer 40 x $10^{-5}$-molaren Lösung der zu untersuchenden Verbindung gegeben. Dies entspricht einer Endkonzentration an Testsubstanz von 10-5 Mol/l. Nach 90 Sekunden Vorinkubationszeit werden 10 µl der P.A.F.-Zubereitung hinzugefügt. Während 4-5 Minuten wird die in den Aggregationsröhrchen auftretende Aggregation mit Hilfe eines computerisierten Aggregometers gemessen.

Die in den nur Blutplättchensuspension enthaltenden Teströhren auftretende Aggregation wird als 0 % gewertet, während die in Blutplättchensuspension und P.A.F.-Zubereitung enthaltenden Teströhrchen auftretende Aggregation als 100 % gewertet wird. Die bei durch Zusatz der Testsubstanzen verursachter Hemmung der durch P.A.F. induzierten Blutplättchenaggregationsverstärkung noch auftretende Aggregation wird gemessen und daraus die erfolgte Aggregationshemmung in % berechnet.

Die nach der vorstehenden Methode mit den Verbindungen der Formel I erhaltenen Ergebnisse werden in der nachfolgenden Tabelle D wiedergegeben.

Tabelle D

| Test. subst. Bsp. Nr. | Anti-P.A.F.-Wirkung in vitro. % Hemmung der P.A.F.-induzierten Aggregation von Blutplättchen aus Kaninchenblut bei einer Testsubstanzkonzentration von $10^{-5}$ Mol/l |
|---|---|
| 14 | 57 |
| 21 | 33 |
| 19 | 72 |

5. In-vitro-Bestimmung der Cyclooxygenase-Hemmung und der 5-Lipoxygenase-Hemmung.

* Tyrodelösung = wäßrige Lösung enthaltend pro Liter 136,9 mMol NaCl, 2,68 mMol KCl, 2,31 mMol $CaCl_2$, 1,0 mMol $MgCl_2$, 11,9 mMol $NaHCO_3$, 1,45 mMol $NaH_2PO_4$ und 5,55 mMol Glucose.

In Zellmembranen enthaltene Arachidonsäure wird nach Aktivierung der Zelle in zweierlei Weise metabolisiert. Unter Einwirkung des Enzyms 5-Lipoxygenase (= 5-LO) werden Leukotriene, unter anderem das Leukotrien $C_4$, und unter Einwirkung des Enzyms Cyclooxygenase (= CO) Prostanoide gebildet. In in-vitro-Systemen werden diese Metaboliten aus der Zelle sekretiert.

Zur Untersuchung der cyclooxygenasehemmenden und der 5-lipoxygenasehemmenden Eigenschaften wird die Hemmwirkung der Testsubstanzen auf die Biosynthese der Arachidonsäurederivate Leukotrien $C_4$ - (= $LTC_4$) und 6-Keto-prostaglandin $F_{1\alpha}$ (= 6-Keto-$PGF_{1\alpha}$) an peritonealen Macrophagenzellen der Maus in vitro bestimmt. Hierzu werden die Gehalte an $LTC_4$ und 6-Keto-$PGF_{1\alpha}$ in einem Kulturmedium von peritonalen Macrophagenzellen der Maus nach Zymosan-Stimulation wie von Scott et al (J. Exp. Med. 152 - (1980), 324-335) und von Fradin et al (Prostaglandins, 33 (1987), 579-589) beschrieben bestimmt.

Eine peritoneale Zellen von männlichen 8 - 10 Wochen alten Mäusen enthaltende Zellsuspension wird auf an sich bekannte Weise gewonnen. Als Zellkulturmedium wird eine unter der Bezeichnung RPMI 1640 im Handel befindliche Lösung (Hersteller Fa. Gibco) verwendet, der Heparin (10 internationale Einheiten/ml) und Antibiotica nach der Rezeptur von Bonney et al. (Biochem. J. 176 (1978) 422-433) zugesetzt werden. Die Zellsuspension wird auf eine Zellkonzentration von $10^6$ Zellen pro ml eingestellt und gleichmäßig auf 24 1-ml-Titerzellen (Wells) enthaltende Titerplatten verteilt. Diese werden zwei Stunden in einem feucht gehaltenen, mit mit 7 % $CO_2$ angereicherter Luft beschickten Inkubator gehalten. Anschließend werden nicht an den Titerzellwänden festhaftende Zellen durch Waschen entfernt. Die verbleibenden an den Wänden anhaftenden Macrophagenzellen werden ca. 12 Stunden in einem Suspensionsmedium, welchem 0,1 % Rinderserumalbumin (BSA = Bovine Serum Albumin) zugesetzt wird, inkubiert. Sodann wird das Suspensionsmedium ersetzt durch eine Salzlösung nach Hanks (= Hanks' Balanced Salt Solution = HBSS) mit 10 mM Hepes (= Hydroxyäthylpiperazinoäthansulfonsäure), welcher eine 0,1 %-ige Lösung der Testsubstanzen in wäßrigem, 1 %-igem Dimethylformamid oder nur das Lösungsmittel zugesetzt worden war. 15 Minuten später wird der Arachidonsäuremetabolismus durch Zugabe von 10 Partikeln Zymosan (= Glykoproteinmischung, isoliert aus Zellwänden von Bierhefe, Saccharomyces cerevisiae, Hersteller Sigma Chemical Co., München) pro Titerzelle stimuliert. Nach 2 Stunden werden Proben der jeweils überstehenden Flüssigkeit auf ihren Gehalt an 6-Keto-$PGF_{1\alpha}$ und $LTC_4$ mittels eines enzymatischen Immunassay (= EIA) untersucht. Dieser EIA wird nach der Methode von Pradelles et al. (Analytical Chem. 57 (1985), 1170-1173) durchgeführt. Die Bestimmung von $LTC_4$ und die Bestimmung von 6-Keto-$PGF_{1\alpha}$ werden jeweils im Vergleich mit einer Vergleichsskala an geeigneten Verdünnungen der Proben (für die $LTC_4$-Bestimmung 1:50 bis 1:250 und für die 6-Keto-$PGF_{1\alpha}$-Bestimmung 1:250 bis 1:1250) durchgeführt. Zur Bestimmung der Hemmwirkung einer $10^{-5}$-molaren Konzentration der Verbindungen wird die Menge an Bezugseicosanoiden bestimmt und daraus die Hemmmwirkung in % Hemmung verglichen mit den Zymosan-Kontrollmessungen berechnet. Die mit diesem Test erhaltenen Ergebnisse werden in der nachfolgenden Tabelle E wiedergegeben.

Tabelle E

| TestSubst. Bsp. Nr. | In vitro % Hemmwirkung in Zymosan-stimulierten peritoenalen Macrophagenzellen der Maus bei einer Konzentration von $10^{-5}$ Mol/l auf die Freisetzung von | |
|---|---|---|
| | 6-Keto-$PGF_{1\alpha}$ | $LTC_4$ |
| 14 | 25 | 10 |
| 20 | 13 | 54 |
| 21 | 11 | 24 |
| 16 | 70 | 97 |
| 1 | 52 | 87 |
| 8 | 40 | 40 |
| 10 | 30 | 37 |
| 3 | 17 | 16 |
| 4 | 71 | 0 |
| 19 | 16 | 57 |

Aufgrund ihrer vorstehend beschriebenen Wirkungen sind die Verbindungen der Formel I als antiinflammatorisch und antiallergisch wirksame Arzneimittel für größere Säugetiere, insbesondere Menschen, geeignet zur Behandlung von entzündlichen und allergischen Erkrankungen. Die erfindungsgemäßen oral wirksamen Verbindungen können in mehrfacher Hinsicht wirken, da sie gegen mehrere der Hauptmediatoren,

welche an entzündlichen Vorgängen und asthmatischen Beschwerden beteiligt sind, wirksam sind.

Aufgrund dieses Wirkungsprofils ist davon auszugehen, daß die erfindungsgemäßen Verbindungen im Rahmen der Behandlung von allergisch bedingten und nicht allergisch bedingten Asthmabeschwerden nicht nur die mit asthmatischen Erkrankunkungen verbundenen symptomatischen Beschwerden lindern, sondern auch die damit verbundene Entzündung mindern können. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoffe enthalten als orale Präparate. Im allgemeinen eignen sich jedoch für Applikationen an größeren Säugetieren, insbesondere Menschen, Arzneiformen mit einem Wirkstoffgehalt von 10 bis 250 mg pro Einzeldosis.

Als Heilmittel können die Verbindungen der Formel I mit üblichen pharmazeutischen Hilfsstoffen in galenischen Zubereitungen, wie z.B. Tabletten, Kapseln, Suppositorien oder Lösungen, enthalten sein. Diese galenischen Zubereitungen können nach an sich bekannten Methoden hergestellt werden unter Verwendung üblicher fester Trägerstoffe wie z.B. Milchzucker, Stärke oder Talkum oder flüssigen Paraffinen und unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Beispiel 1:

2-{4-[4-(N-(1-(4-Fluorphenylmethyl)-1H-benzimidazol-2-yl)-amino)-piperidin-1-yl]-butyl}-2H-1,4-benzothiazin-3(4H)-on.

A) 45,3 g (= 0,23 m) 6-Bromcapronsäure wurden unter Rühren mit 63,6 g (= 0,235 m) Phosphortribromid versetzt und anschließend wurden 75,1 g (=0,047 m) Brom zugegeben, wobei die erste Hälfte tropfenweise und der Rest schneller zugegeben wurden. Die Mischung wurde dann auf eine Temperatur von 85-90 °C erhitzt und 1 1/2 Stunden auf dieser Temperatur gehalten. Sodann wurden bei dieser Temperatur nochmals 18,4 g (= 0,115 m) Brom zugegeben und das Reaktionsgemisch weitere 18 Stunden auf einer Temperatur von 85-90 °C gehalten. Zur Aufarbeitung wurde das Reaktionsgemisch anschließend auf 20 °C gekühlt und in ein Gemisch aus 700 ml gekühltem Wasser und 500 ml kaltem Hexan gegeben. Die organische Phase wurde abgetrennt und die wäßrige Phase noch zweimal mit je 100 ml Hexan gewaschen. Die organischen Phasen wurden vereinigt und über Natriumsulfat getrocknet. Sodann wurde das Lösungsmittel abdestilliert. Das als öliger Rückstand verbleibende 2,6-Dibromcapronsäurebromid wurde durch IR- und NMR-Spektroskopie charakterisiert und ohne weitere Reinigung weiterverarbeitet.

B) 9,25 g (= 0,07 m) 2-Aminothiophenol, 27,9 g Benzyltrimethylammoniumchlorid und 25 g Natriumhydrogencarbonat wurden unter Rühren in 150 ml Chloroform gegeben. Die erhaltene Suspension wurde auf -5 °C gekühlt und zu der gekühlten Suspension wurde eine Lösung von 25,3 g 2,6-Dibromcapronsäurebromid in 70 ml Chloroform langsam unter Rühren zugefügt. Die Zugabe benötigte 30 min. und die Temperatur schwankte zwischen -5 °C und +5 °C. Die Mischung wurde für eine weitere Stunde in diesem Temperaturbereich gehalten. Anschließend wurde die Mischung 7 Std. lang unter Rückfluß erhitzt. Sodann wurde abgekühlt, filtriert und aus dem erhaltenen Filtrat das Chloroform abdestilliert. Der verbleibende Rückstand wurde mit Wasser und Toluol versetzt. Die organische Phase wurde abgetrennt, getrocknet und durch Säulenchromatographie fraktioniert. Die 2-(4-Brombutyl)-2H-1,4-benzothiazin-3(4H)-on enthaltende Fraktion wurde abgetrennt. Hieraus wurden nach Kristallisation in Gegenwart von Diäthyläther 5,9 g des 2-(4-Brombutyl)-2H-1,4-benzothiazin-3(4H)-on als leicht beige gefärbte Kristalle mit einem Schmelzpunkt von 100 C erhalten.

C) Eine Mischung aus 3,3 g (= 0,011 m) 2-(4-Brombutyl)-2H-1,4-benzothiazin-3(4H)-on, 3g (= 0,0925 m) N-[1-(4-Fluorphenylmethyl)-1H-benzimidazol-2-yl]-N-(piperidin-4-yl)-amin und 2,02 g Triäthylamin in einem Gemisch aus 50 ml Toluol und 5 ml Dimethylformamid wurde unter Rühren 11 Stunden am Rückfluß erhitzt.

Zur Aufarbeitung wurde das Reaktionsgemisch angesäuert, das Lösungsmittel abdestilliert und der Rückstand wurde mit Dichlormethan und wäßriger Natriumhydrogencarbonatlösung versetzt. Die organische Phase wurde abgetrennt und durch Abdestillieren des Lösungsmittels eingeengt. Der verbleibende Rückstand wurde durch fraktionierte Säulenchromatographie gereinigt. Die rohe Titelverbindung wurde als amorpher Feststoff erhalten. Dieser wurde in Isopropanol gelöst. Der isopropanolischen Lösung der Titelverbindung wurde isopropanolische 2,3 N Salzsäure zugesetzt, wobei das Dihydrochlorid der Titelverbindung auskristallisierte. Es wurden 1,3 g 2-{4-[4-(N-(1-(4-Fluorphenylmethyl)-1H-benzimidazol-2yl)-amino)-piperidin-1-yl]-butyl}-2H-1,4-benzothiazin-3(4H)-on-dihydrochlorid in Form farbloser Kristalle mit einem Schmelzpunkt von 206 °C erhalten.

Beispiel 2:

2-[3-(4-Benzylpiperidin-1-yl)-propyl]-2H-1,4-benzoxazin-3(4H)-on.

5,6 g (= 0,02 m) 2-(3-Brompropyl)-2H-1,4-benzoxazin-3(4H)-on (hergestellt ausgehend von 2-Aminophenol und 2,5-Dibrompentanoylbromid analog Beispiel 1 B), 3,86 g (=0,022 m) 4-Benzylpiperidin und 4,05 g Triäthylamin wurden nacheinander in 70 ml Toluol gegeben. Das Reaktionsgemisch wurde 7 Stunden unter Rühren am Rückfluß erhitzt. Zur Aufarbeitung wurde das Reaktionsgemisch abgekühlt, und es wurden 100 ml wäßriger 20 %-iger Salzsäurelösung zugesetzt. Es bildete sich eine ölige Paste, welche abgetrennt, mit Toluol gewaschen und dann in einer Mischung aus einer wäßrigen Natriumhydrogencarbonatlösung und Dichlormethan suspendiert wurde. Die organische Phase wurde abgetrennt, über Natriumsulfat getrocknet und dann das Lösungsmittel abdestilliert. Der verbleibende ölige Rückstand wurde durch Säulenchromatographie fraktioniert. Die so erhaltene Titelverbindung wurde aus 15 ml Essigsäureäthylester kristallisiert. Es wurden 6 g 2-[3(4-Benzylpiperidin-1-yl)-propyl]-2H-1,4-benzoxazin-3(4H)-on als weiße Kristalle mit einem Schmelzpunkt von 108 °C erhalten.

Beispiel 3:

2-[3-(4-Benzylpiperidin-1-yl)-propyl]-2H-1,4-benzoxazin-3(4H)-thion.

3 g (= 0,0082 m) 2-[3-(4-Benzylpiperidin-1-yl)-propyl]-2H-1,4-benzoxazin-3(4H)-on (Herstellung s. Beispiel 2) und 3,91 g $P_4S_4$ wurden in 50 ml Xylol unter Rühren eine Stunde am Rückfluß erhitzt. Zur Aufarbeitung wurde abgekühlt, der dabei gebildete Niederschlag wurde abgetrennt und in eine Mischung aus 100 ml 2 N Natriumhydroxidlösung und 150 ml Dichlormethan gegeben. Die organische Phase wurde abgetrennt und das Lösungsmittel abdestilliert. Die als Rückstand verbleibende rohe Titelverbindung wurde durch Säulenchromatographie gereinigt. Die so erhaltene Titelverbindung wurde in Isopropanol gelöst. Durch Zusatz von isopropanolischer 2,3 N Salzsäurelösung wurde das Dihydrochlorid der Titelverbindung gefällt. Es wurden 1,3 g 2-[3-(4-Benzylpiperidin-1-yl)-propyl]-2H-1,4-benzoxazin-3(4H)-thion-dihydrochlorid mit einem Schmelzpunkt von 210 °C gewonnen.

Beispiel 4:

2-[4-(4-Fluorbenzoyl)-piperidin-1-yl]-2H-1,4-benzothiazin-3 (4H)-on.

A) Zu einer Suspension von 10,0 mMol 2H-1,4-Benzothiazin-3(4H)-on in 10 ml Dichlormethan wurden unter Rühren 0,8 ml (= 1 Äquivalent) Sulfonylchlorid tropfenweise unter Rühren bei Raumtemperatur gegeben, und das Reaktionsgemisch wurde noch weitere 5 Stunden bei Raumtemperatur gerührt. Zur Aufarbeitung wurde das Gemisch unter vermindertem Druck zur Trockne eingedampft. Das als Rückstand verbleibende 2-Chlor-2H-1,4-benzothiazin-3(4H)-on (Schmelzpunkt 189 - 210 °C unter Zersetzung) wurde ohne weitere Reinigung in der nächsten Stufe weiter verarbeitet.

B) 7 g (= 0,035 m) 2-Chlor-2H-1,4-benzothiazin-3(4H)-on, 7,25 g (= 0,035 m) 4-(4-Fluorbenzoyl)-piperidin und 7,1 g Triäthylamin wurden in 200 ml Toluol 4 Stunden unter Rühren am Rückfluß erhitzt. Zur Aufarbeitung wurde das Reaktionsgemisch abgekühlt, und dann wurden 200 ml Toluol und 200 ml Wasser zugefügt. Der pH-Wert des Gemisches wurde durch Zugabe von 0,1 N Salzsäurelösung auf 7 eingestellt. Die organische Phase wurde abgetrennt, mit 200 ml Wasser gewaschen, und das Lösungsmittel wurde abdestilliert. Aus dem verbleibenden öligen Rückstand wurde die Titelverbindung durch fraktionierte Säulenchromatographie gewonnen, aus Essigsäureäthylester kristallisiert und nochmals in einem Gemisch aus Äthanol/Diäthyläther umkristallisiert. Es wurden 1,95 g 2-[4-(4-Fluorbenzoyl)-piperidin-1-yl]-2H-1,4-benzothiazin-3(4H)-on als farbloses kristallines Pulver mit einem Schmelzpunkt von 218 °C erhalten.

Beispiel 5:

4-Methyl-2-[4-(4-phenyl-1,2,3,6-tetrahydropyridin-1-yl)-butyl]-2H-1,4-benzothiazin-3(4H)-on.

2,4 g (= 0,0076 m) 2-(4-Brombutyl)-4-methyl-2H-1,4-benzothiazin-3(4)-on, 1,49 g (=0,0076 m) 4-Phenyl-1,2,3,6-tetrahydropyridin-hydrochlorid und 3,09 g Triäthylamin wurden in 20 ml Toluol gegeben. Die erhaltene Suspension wurde unter Rühren 10,5 Stunden erhitzt. Zur Aufarbeitung wurde abgekühlt und die Suspension in 30 ml 20 %-ige wäßrige Salzsäurelösung gegeben. Der gebildete Niederschlag wurde abfiltriert, und die wäßrige Phase wurde alkalisch gestellt und mit 200 ml Dichlormethan extrahiert. Der Niederschlag wurde in alkalisiertes Wasser gegeben und mit 450 ml Dichlormethan extrahiert. Die vereinigten Dichlormethanextrakte wurden gewaschen, über Natriumsulfat getrocknet und das Lösungsmittel abdestilliert. Es wurden 2,1 g 4-Methyl-2-[4-(4-phenyl-1,2,3,6-tetrahydropyridin-1-yl)-butyl]-2H-1,4-benzothiazin-3-(4H)-on als beigefarbenes Pulver erhalten.

Beispiel 6:

4-Methyl-2-[4-(4-phenyl-1,2,3,6-tetrahydropyridin-1-yl)-butyl]-2H-1,4-benzothiazin-3(4H)-on.

1,77 g (= 0,004 m) 2-[4-(4-Phenyl-1,2,3,6-tetrahydropyridin-1-yl)-butyl]-2H-1,4-benzothiazin-3(4H)-on ( s. Beispiel 10, Herstellung analog Beispiel 1) wurden in 20 ml wasserfreiem Dimethylformamid unter Rühren gelöst. Unter Stickstoffatmosphäre wurden 0,121 g Natriumhydrid zugegeben und die gebildete Suspension wurde 10 min. auf Raumtemperatur gehalten. Dann wurde auf einmal 0,70 g Methyljodid zugegeben und das Reaktionsgemisch wurde 2,5 Std. bei Raumtemperatur gerührt. Zur Aufarbeitung wurde das Lösungsmittel abdestilliert, zu dem Rückstand 100 ml Wasser gegeben und mit 100 ml Äthylacetat extrahiert. Die organische Phase wurde durch Eindampfen des Lösungsmittels konzentriert, und die als Rückstand verbleibende rohe Titelverbindung wurde durch Säulenchromatographie gereinigt. Es wurden 0,6 g 4-Methyl-2-[4-(4-phenyl-1,2,3,6-tetrahydropyridin-1-yl)-butyl]-2H-1,4-benzothiazin-3(4H)-on als beigefarbenes Pulver erhalten.

Beispiel 7:

7-Hydroxy-2-{4-[4-(4-fluorbenzoyl)-piperidin-1-yl]-butyl}-2H-1,4-benzothiazin-3(4H)-on.

675 mg (= 0,0015 m) 7-Methoxy-2-{4-[4-(4-fluorbenzoyl)-piperidin-1-yl]-butyl}-2H-1,4-benzothiazin-3-(4H)-on (s. Beispiel 18, Herstellung analog Beispiel 4) wurden in 5 ml Dichlormethan unter Feuchtigkeitsausschluß gegeben. Nach Abkühlen auf -5 °C wurde eine Lösung von 1,10 g Bortribromid in 1 ml Methylenchlorid tropfenweise unter Rühren zugefügt. Anschließend wurde noch weitere 30 min. bei Raumtemperatur gerührt. Zur Aufarbeitung wurde das Reaktionsgemisch unter Rühren zu einer Mischung aus Eis und wäßriger Natriumhydrogencarbonatlösung gegeben. Dann wurden 200 ml Chloroform zugefügt. Die gebildete organische Lösung wurde mit Wasser gewaschen, über Natriumsulfat getrocknet, filtriert und unter vermindertem Druck eingeengt. Aus dem verbleibenden Rückstand wurde die Titelverbindung durch fraktionierte Säulenchromatographie gewonnen. Die die Titelverbindung enthaltenden Fraktionen wurden eingeengt und mit Äther versetzt. Es wurden 55 mg 7-Hydroxy-2-{4-[4-(4-fluorbenzoyl)-piperazin-1-yl]-butyl}-2H-1,4-benzothiazin-3(4H)-on erhalten.

Nach den in den vorstehenden Beispielen beschriebenen Verfahren wurden auch die in der folgenden Tabelle I aufgeführten Verbindungen der Formel I erhalten.

Tabelle I

| Bsp. | $R^1$ | $R^2$ | $R^3$ | X | Y | n | $R^4$ | $R^5$ | $R^6$ | Salzform | Fp in °C |
|---|---|---|---|---|---|---|---|---|---|---|---|
| 8 | H | H | H | S | O | 3 | H | H | im-NH- | $H_2O \rightarrow H_2O$ | 220 |
| 9 | H | H | H | O | O | 4 | H | H | ph- | Base | 160 |
| 10 | H | H | H | S | O | 4 | bin | | ph- | HCl | 220 |
| 11 | H | H | H | S | O | 4 | H | OH | ph- | Base | 138 |
| 12 | H | H | H | O | O | 4 | bin | | ph- | Base | 148 |
| 13 | H | H | H | O | O | 4 | H | OH | ph- | HCl | 176 |
| 14 | H | 6-Cl | H | O | O | 3 | H | CN | ph- | Base | 131 |
| 15 | H | H | H | S | O | 4 | H | CN | ph- | Base | 154 |
| 16 | H | H | H | S | O | O | H | H | $ph-CH_2-$ | Base | 145 - 148 |
| 17 | H | 7-F- | H | S | O | 4 | bin | | ph- | Base | 152 |
| 18 | H | $7-CH_3O-$ | H | S | O | 4 | H | H | 4-F-pH-CO- | Base | am |
| 19 | H | $6-CH_3-$ | $7-CH_3-$ | S | O | 4 | bin | | ph- | Base | 153 |
| 20 | H | 7-Cl | H | S | O | 4 | H | H | im-NH- | 2.HCl | 160 |
| 21 | H | H | H | S | O | 3 | H | H | $ph-CH_2-$ | Base | 114 |

im = 1-(4-Fluorphenylmethyl)-1H-benzimidazol-2-yl, ph = Phenyl,
bin = Bindung, HCl= Hydrochlorid, Base = freie Base, am = amorph.

Beispiel I:

2-{4-[4-(N-(1-(4-Fluorphenylmethyl)-1H-benzimidazol-2-yl)-amino)-piperidin-1-yl]-butyl}-2H-1,4-benzothiazin-3(4H)-on enthaltende Tabletten.

Man stellte Tabletten in folgender Zusammensetzung pro Tablette her:

| | |
|---|---|
| 2-{4-[4-(N-(1-(4-Fluorphenylmethyl)-1H-benzimidazol-2-yl)-amino)-piperidin-1-yl]-butyl}-2H-1,4-benzothiazin-3(4H)-on | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |
| und sodann zu Tabletten von 240 mg verpreßt. | |

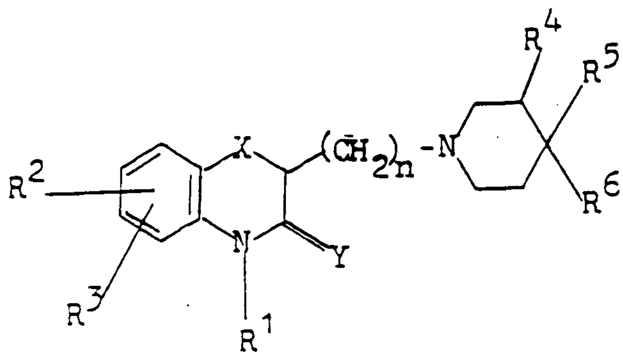

I

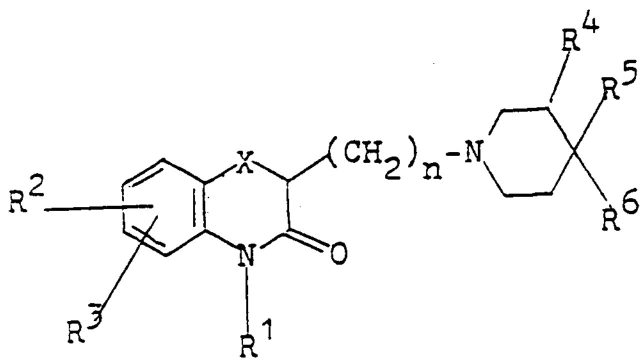

Ia

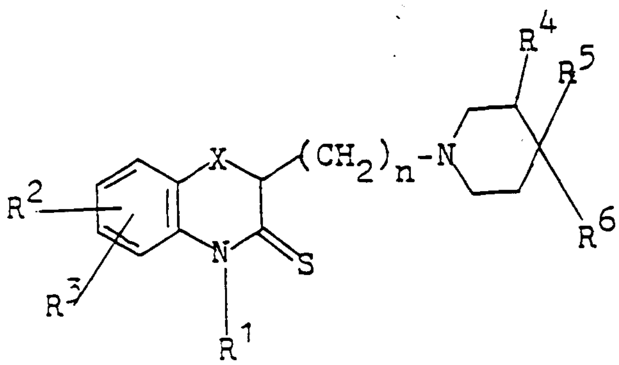

Ib

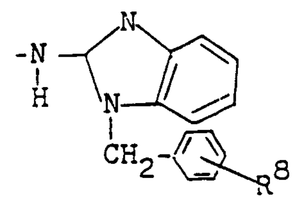

a

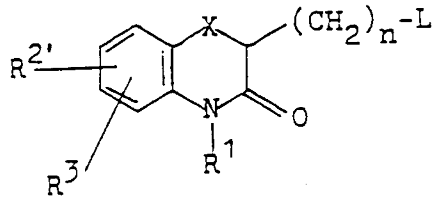

II

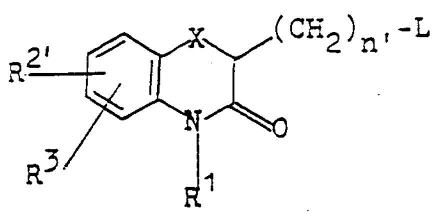

IIa

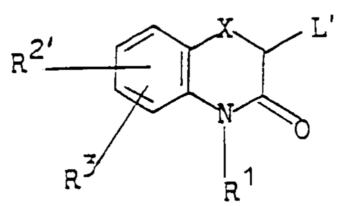

IIb

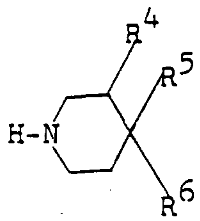

III

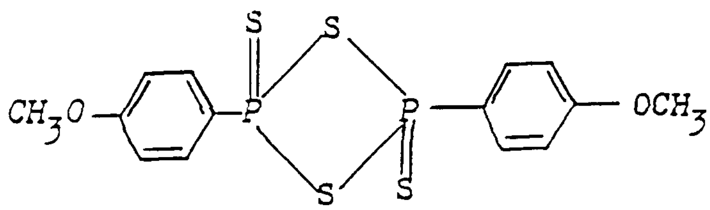

IV

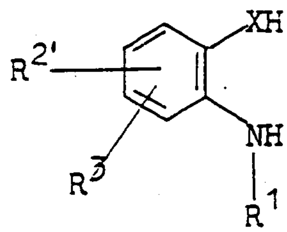

V

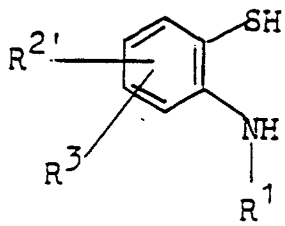

Va

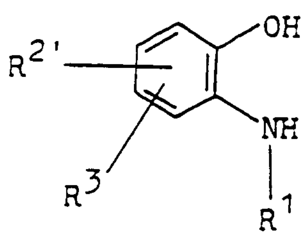

Vb

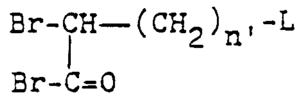

VI

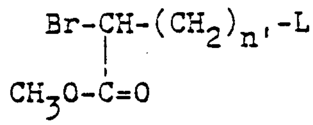

VII

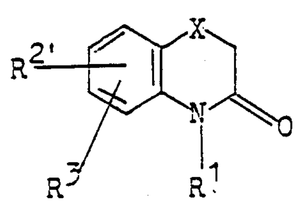

VIII

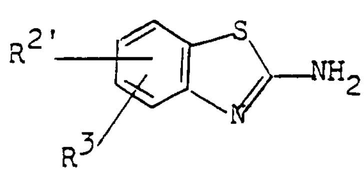

IX

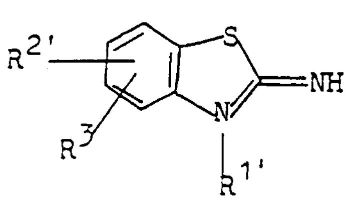

X

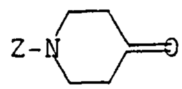

XI

$R^6$ Mg-L'

XII

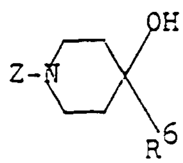

XIII

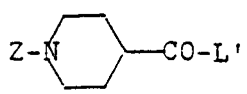

XIV

H-$R^7$

XV

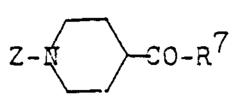

XVI

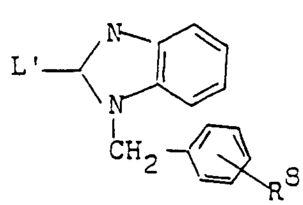

XVII

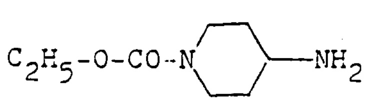

XVIII

## Patentansprüche

**1.** Verbindungen der allgemeinen Formel I

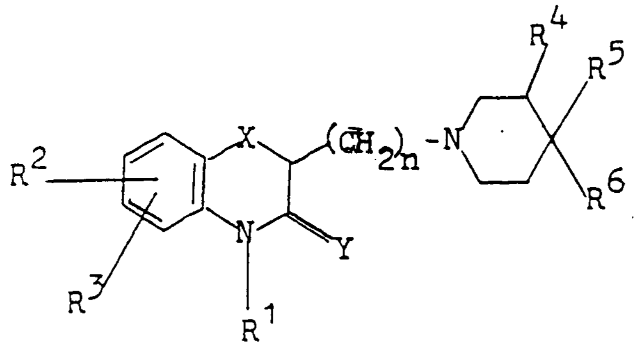

worin

| | |
|---|---|
| X | Sauerstoff oder Schwefel bedeutet, |
| Y | Sauerstoff oder Schwefel bedeutet, |
| $R^1$ | Wasserstoff oder niederes Alkyl bedeutet, |
| $R^2$ | Wasserstoff, niederes Alkyl, Halogen, niederes Alkoxy, Hydroxy, Nitro oder Trifluormethyl bedeutet, und |
| $R^3$ | Wasserstoff, niederes Alkyl, Halogen oder niederes Alkoxy bedeutet, oder |
| $R^2$ und $R^3$ | an benachbarte Kohlenstoffatome gebunden sind und gemeinsam eine Alkylendioxygruppe mit 1-2 Kohlenstoffatomen bedeuten, |
| n | für eine ganze Zahl von 0 bis 4 steht, |
| $R^4$ | Wasserstoff bedeutet, und |
| $R^5$ | Wasserstoff, Hydroxy oder Cyano bedeutet, oder |
| $R^4$ und $R^5$ | gemeinsam eine Bindung bilden, |
| $R^6$ | für eine A-$R^7$-Gruppe steht, worin $R^7$ eine gegebenenfalls durch 1-3 Substituenten aus der Gruppe niederes Alkyl, niederes Alkoxy, Halogen und Trifluormethyl substituierte Phenylgruppe bedeutet, und A eine -$CH_2$-Gruppe, eine-CO-Gruppe oder eine Bindung bedeutet, oder |
| $R^6$ | für die Gruppe der Formel a |

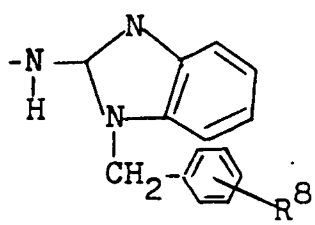

worin $R^8$ Halogen bedeutet, steht,

und deren physiologisch verträgliche Säureadditionssalze.

**2.** Verbindungen gemäß Anspruch 1, dadurch gekennzeichnet, daß Y Sauerstoff bedeutet.

**3.** Verbindungen nach einem der vorhergehenden Ansprüche, dadurch gekennzeichnet, daß n für 3 oder 4 steht.

**4.** Verbindungen nach einem der vorhergehenden Ansprüche, dadurch gekennzeichnet, daß $R^1$ Wasserstoff bedeutet.

**5.** Verbindungen nach einem der vorhergehenden Ansprüche, dadurch gekennzeichnet, daß $R^2$ Wasserstoff oder niederes Alkyl und $R^3$ Wasserstoff bedeuten.

**6.** Verbindungen nach einem der vorstehenden Ansprüche, dadurch gekennzeichnet, daß $R^4$ Wasserstoff bedeutet.

**7.** Verbindungen gemäß Anspruch 1, dadurch gekennzeichnet, daß X Sauerstoff oder Schwefel und Y Sauerstoff bedeuten, $R^1$ Wasserstoff bedeutet, $R^2$ Wasserstoff oder niederes Alkyl bedeutet, $R^3$ Wasserstoff bedeutet, n für 3 oder 4 steht, $R^4$ und $R^5$ je Wasserstoff bedeuten oder gemeinsam eine Bindung bilden und $R^6$ für einen gegebenenfalls durch niederes Alkyl, niederes Alkoxy oder Halogen substituierten Benzyl-, Benzoyl- oder Phenylrest oder für die Gruppe a steht.

**8.** Verbindungen gemäß Anspruch 7, worin X Schwefel bedeutet, $R^2$, $R^4$ und $R^5$ je Wasserstoff bedeuten und $R^6$ die 1-(4-Fluorphenylmethyl)-1H-benzimidazol-2-ylgruppe bedeutet.

**9.** Arzneimittel, enthaltend eine pharmakologisch wirksame Menge einer Verbindung gemäß Anspruch 1 und übliche pharmazeutische Hilfs- und/oder Trägerstoffe.

**10.** Verfahren zur Herstellung von Verbindungen der allgemeinen Formel I

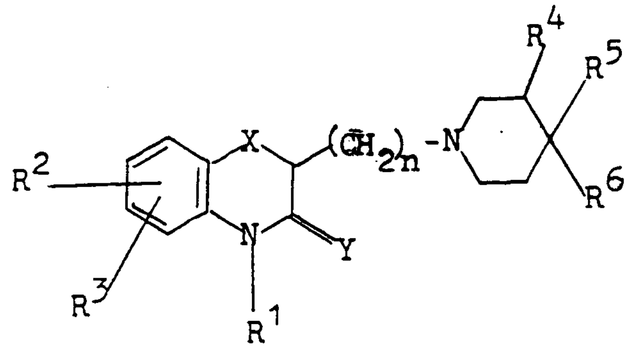

worin

| | |
|---|---|
| X | Sauerstoff oder Schwefel bedeutet, |
| Y | Sauerstoff oder Schwefel bedeutet, |
| $R^1$ | Wasserstoff oder niederes Alkyl bedeutet, |
| $R^2$ | Wasserstoff, niederes Alkyl, Halogen, niederes Alkoxy, Hydroxy, Nitro oder Trifluormethyl bedeutet, und |
| $R^3$ | Wasserstoff, niederes Alkyl, Halogen oder niederes Alkoxy bedeutet, oder |
| $R^2$ und $R^3$ | an benachbarte Kohlenstoffatome gebunden sind und gemeinsam eine Alkylendioxygruppe mit 1-2 Kohlenstoffatomen bedeuten, |
| n | für eine ganze Zahl von 0 bis 4 steht, |
| $R^4$ | Wasserstoff bedeutet, und |
| $R^5$ | Wasserstoff, Hydroxy oder Cyano bedeutet, oder |
| $R^4$ und $R^5$ | gemeinsam eine Bindung bilden, |
| $R^6$ | für eine A-$R^7$-Gruppe steht, worin $R^7$ eine gegebenenfalls durch 1-3 Substituenten aus der Gruppe niederes Alkyl, niederes Alkoxy, Halogen und Trifluormethyl substituierte Phenylgruppe bedeutet, und A eine $-CH_2$-Gruppe eine-CO-Gruppe oder eine Bindung bedeutet, oder |
| $R^6$ | für die Gruppe der Formel a |

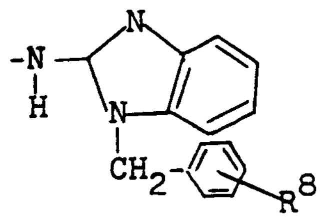

worin $R^8$ Halogen bedeutet, steht,

und deren Säureadditionssalzen, dadurch gekennzeichnet, daß man

a) zur Herstellung von Verbindungen der allgemeinen Formel Ia

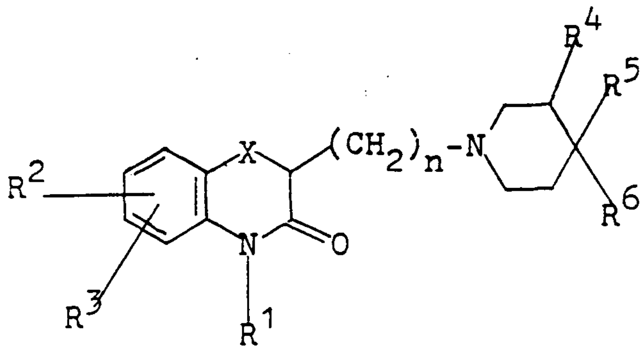

worin X, $R^1$, $R^2$, $R^3$, n, $R^4$, $R^5$ und $R^6$ obige Bedeutung besitzen, Verbindungen der allgemeinen Formel II

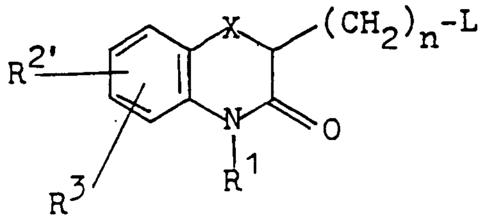

worin X, $R^1$, $R^3$ und n obige Bedeutung besitzen, und $R^{2'}$ die für $R^2$ angegebene Bedeutung besitzt, wobei jedoch eine Hydroxygruppe durch eine nachträglich abspaltbare Schutzgruppe geschützt ist, und L für einen aminolytisch abspaltbaren Rest, insbesondere Halogen, steht, umsetzt mit Verbindungen der allgemeinen Formel III

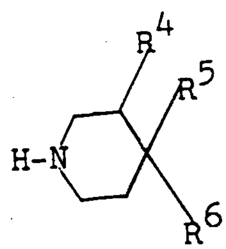

worin $R^4$, $R^5$ und $R^6$ obige Bedeutung besitzen, und anschließend eine allfällige Hydroxyschutzgruppe wieder abspaltet oder

b) Verbindungen der allgemeinen Formel Ia in Verbindungen der allgemeinen Formel Ib

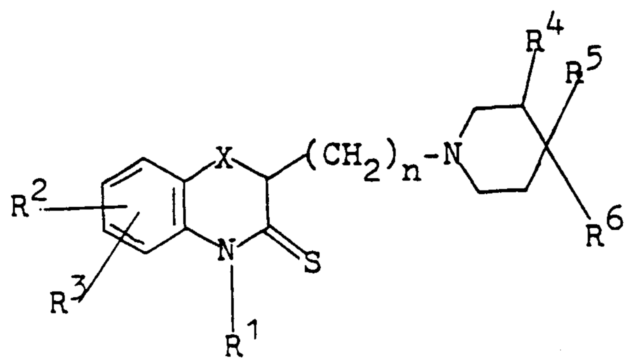

worin X, $R^1$, $R^2$, $R^3$, n, $R^4$, $R^5$ und $R^6$ obige Bedeutung besitzen, überführt und gewünschtenfalls erhaltene Verbindungen der allgemeinen Formel I, worin $R^1$ Wasserstoff bedeutet, zu Verbindungen der allgemeinen Formel I, worin $R^1$ niederes Alkyl bedeutet, alkyliert und/oder in erhaltenen Verbindungen der allgemeinen Formel I, worin $R^2$ Methoxy bedeutet, die Methoxygruppe zur Hydroxygruppe spaltet, und gewünschtenfalls freie Verbindungen der Formel I in ihre Säureadditionssalze überführt oder die Säureadditionssalze in die freien Verbindungen der Formel I überführt.